# EUROPEAN PATENT SPECIFICATION

(11) **EP 2 531 039 B1**
(45) Date of publication and mention of the grant of the patent: **06.12.2017**
(21) Application number: 11702026.3
(22) Date of filing: 01.02.2011
(51) Int. Cl.: A23C 9/12, A23J 3/10, C12N 9/24

(54) **Use of sialidase in dairy technology**
Verwendung von Sialidase in der Molkereitechnologie
Utilisation de la sialidase dans la téchnologie laitière

(30) Priority: 03.02.2010 EP 10152527
(43) Date of publication of application: 12.12.2012
(73) Proprietor: DSM IP Assets B.V., 6411 TE Heerlen (NL)
(72) Inventor: DEKKER, Petrus, Jacobus, Theodorus, 2497 AE Den Haag (NL); METSELAAR, Ronald, 2901 HD Capelle A/d Ijssel (NL); SEIN, Arjen, 2316 AA Leiden (NL)
(74) Representative: DSM Intellectual Property
(86) International application number: PCT/EP2011/051391
(87) International publication number: WO 2011/095477

(56) References cited:
- EP-A1- 0 349 666
- EP-A2- 0 283 675
- WO-A1-02/074097
- WO-A1-2007/060288
- WO-A2-03/049547
- WO-A2-2008/101893
- LORENZEN P C: "Enzymatische Quervernetzung von Milcheiweiss", DEUTSCHE MILCHWIRTSCHAFT, HILDESHEIM, DE, vol. 53, no. 17, 21 August 2002 (2002-08-21), pages 726-728, XP008083031, ISSN: 0012-0480
- AHMAD F ET AL: "The intrinsic viscosity of glycoproteins", INTERNATIONAL JOURNAL OF BIOCHEMISTRY, PERGAMON LNKD- DOI:10.1016/0020-711X(80)90240-2, vol. 11, no. 2, 1 January 1980 (1980-01-01), pages 91-96, XP023406530, ISSN: 0020-711X [retrieved on 1980-01-01]

## Description

### Field of the invention

The invention relates to the use of sialidase in stirred, set or drinking yoghurt. Sialidase can for example be used to improve the texture of a dairy product or to reduce syneresis.

### Background of the invention

Conventionally, sour dairy preparations have been manufactured by acidifying milk with an acidifying agent specific to each product at a suitable temperature. Fermented dairy products are obtained by incubating milk or a raw material derived from milk with particular micro-organisms such as lactic acid bacteria. The raw material is often cow's milk, but the milk of other animals such as buffalos, horses, camels, sheep or goats can also be used, as can cream or whey. The milk may be whole milk, but also partially or completely skimmed milk.

As a non-limiting example, yoghurt is described in more detail.

Traditionally, yoghurt is produced by inoculation of milk with *Lactobacillus delbrueckii subsp. bulgaricus* and *Streptococcus thermophilus* as starter cultures. It is a traditional method to preserve milk through acidification. Before acidifying, necessary raw materials (such as sweetener, flavouring agents and texturizers) can be added to the milk, and the milk is then typically pasteurized and homogenized. The milk is acidified to a pH specific to each product. Three basic types of yoghurt exist, according to its physical state in the retail container: set yoghurt, stirred yoghurt and drinking yoghurt. Set yoghurt is fermented after being packed in a retail container, and stirred yoghurt is almost fully fermented in a fermentation tank before it is packed, the yoghurt gel being broken up during the stirring and pumping. Drinking yoghurt is a variant on stirred yoghurt, where the gel breaking step is more severe to yield a liquid, drinkable product that often is further stabilised colloidally by the use of pectins. Quite often a process for drinking yoghurt involves the mixing of a second aqueous phase to the stirred yoghurt, containing for instance flavours, sweeteners, hydrocolloids, fruit juice and so on. All types of yoghurt undergo the phenomenon of water separation (syneresis).

Conventionally, the dry substance content of milk is increased or decreased for adjusting the texture of sour dairy preparations. The dry substance content can be increased by removal of water by evaporating or ultra- or nano-filtration from the milk or by the use of powders. The protein content of the raw material, for example milk, affects the texture of the end product in a manner allowing the texture of the sour dairy preparation, such as a soured fresh dairy product to be modified thicker or runnier by increasing or decreasing the protein content. The protein content of the raw material, for example milk, can be increased by removal of water by evaporation or by the use of protein powder addition. The protein powder may originate from milk, such as milk powder, whey protein powder or casein protein powder, but proteins not originating from milk are also usable. Fortification of the milk using these methods is especially useful when a sour dairy preparation, like yoghurt, is produced from skimmed or defatted milk. Since the fat content has a large influence on the texture of the final product, low-fat yoghurts produced without any additions are thin. Since market preference is on highly structured sour dairy preparations, it is advantageous to fortify the milk before acidification. Evaporation or the use of protein powder addition, however, increases cost price of the final sour dairy preparation. Furthermore, high concentrations of total solids added to the milk before fermentation may result in conditions inhibitory to bacterial growth, leading to long fermentation times and poor acid development.

One of the measures frequently used for product stability and improved thickening is the addition of stabilizers or texturizers (chemically modified starch, native starch, carrageenan, guar gum, pectin, gelatin, etc.)(Everett, D. W., & McLeod, R. E. (2005) Int. Dairy J. 15, 1175-1183). However, those food additives may adversely affect the true taste and aroma of yoghurt. In addition, the use of those hydrocolloids results in a non-natural image, and it is not allowed in all countries. Hence, in the latter case yoghurt manufacturers have to make use of different technologies to guarantee an acceptable texture of the end-products. As the structure of protein aggregates in drinking yoghurt is so severely damaged, hydrocolloids such as pectins are usually added to colloidally stabilise the protein flocs to form a homogeneous and uniform product.

The use of yoghurt starter cultures that contain strains that produce exopolysaccharides (*S. thermophilus, Lb. delbrueckii subsp. bulgaricus* or both) is a promising alternative for the non-dairy texturisers as mentioned above. Exopolysaccharides from yoghurt bacteria are microbial polysaccharides, intracellular synthesized and extracellular secreted, which may function as texture-forming constituents. Exopolysaccharides that are produced in situ, i.e. through inoculation of milk with a yoghurt starter culture that contains exopolysaccharide-producing strains, have the capacity to retain water and hence avoid syneresis, to improve the viscosity and hence guarantee a good final texture, and to replace fat without affecting the mouth feel when the yoghurt is eaten. However, exopolysaccharide production in milk by thermophilic lactic acid bacteria such as *Streptococcus thermophilus* is low and unstable when carried out using the traditional batch process technologies for the production of yoghurt. Additionally, lactic acid bacteria producing excessive amounts of exopolysaccharides are often poor in acidification. Therefore, the yoghurt-making process is slowed which is not desirable in an industrial process.

In the past, enzyme-assisted processes to increase the structure of sour dairy preparations have been described. The use of a texture-modifying transglutaminase enzyme in the manufacture of dairy preparations, particularly sour dairy preparations, is known. In sour dairy preparations, such as soured fresh dairy products (yoghurt, set-type yoghurt, viili, fermented milk), the use of a transglutaminase enzyme serves to harden the texture, modify the texture to be finer, and to reduce syneresis. WO2007/060288 and references therein describes the use of transglutaminase during the acidification of a dairy product. A major disadvantage of the use of transglutaminase in the production of sour dairy preparations is its high cost. Since transglutaminase is difficult to produce, the cost price of this enzyme is high.

Despite all efforts to modify the texture of sour dairy preparations, especially low fat preparations, no satisfactory method has been developed yet that could reduce the common use of the expensive skim milk powder addition in the preparation of sour dairy preparations. Moreover, no satisfactory method has been developed for improving a characteristic of current sour dairy preparations (i.e. without reducing the common use of expensive texture improving additives), such as a soured dairy product like stirred yoghurt, set yoghurt or drink yoghurt.

### Summary of the invention

It has now surprisingly been found that the addition of a sialidase enzyme in the process of making a yoghurt or drinking yoghurt allows a reduction in the protein content or allows a reduction in the amount of used stabilizers or texturizers (such as pectin), without any damage to or adverse effect on the texture of the yoghurt or drinking yoghurt. Therefore, the use of sialidase can reduce the addition of extra protein without affecting the final texture Alternatively the firmness or viscosity can be increased or the syneresis can be decreased by the addition of a sialidase enzyme. Sialidase can be added to the milk and incubated at a suitable temperature, before an optional heat treatment. Alternatively, sialidase can be added after the optional heat treatment of the milk, during fermentative acidification Sialidase may also be used to pre-treat a fraction of the yoghurt or drinking yoghurt, or additional dairy protein, which is added during the making of the yoghurt or drinking yoghurt

### Description of Figures

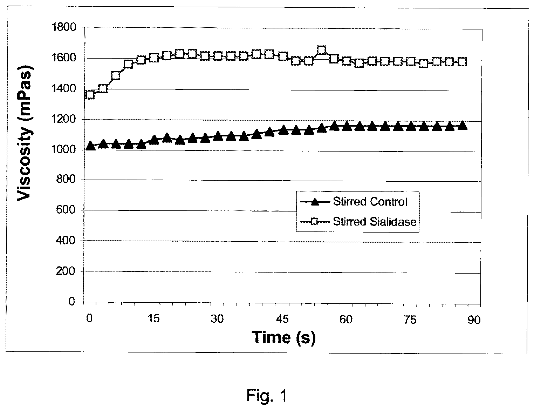

Figure 1: Brookfield measurements after 1 week for stirred yoghurt. Yoghurt treated with sialidase (dots) compared to untreated control yoghurt (triangles).

### Detailed description of the invention

κ-casein is part of the casein micelles in milk and is a glycoprotein that contains sialic acid residues at known positions (Cases et al, J Food Sci (2003) 68, 2406-2410). It is known that there is a micro-heterogeneity in the population of κ-casein due to differences in glycosylation and sialic acid content (Robitaille et al, Food Res Int (1995) 28, 17-21). Several studies describe the effect of removal of sialic acid residues using the sialidase derived from *Clostridium perfringens.* Parameters that were examined are the stability of the casein micelle against heat and chymosin degradation. Gibbons et al (Biochim Biophys Acta (1962) 56, 354-356) used solutions of purified κ-casein and showed that removal of sialic acid residues does not affect the action of chymosin on κ-casein. Vreeman et al (Biochem J (1986) 240, 87-97) compared the kinetic behaviour of chymosin towards purified κ-casein fractions with or without glycosylation and found that the deglycosylated κ-casein is a better substrate for chymosin. Also, Minkiewicz et al (Pol J Food Nutr Sci (1993), 243, 39-48) using an artificial reconstituted casein micelle system, showed that sialic acid residues are contributing to the heat stability of casein micelles, which was later confirmed by Robitaille et al (Food Res Int (1995) 28, 17-21). In a separate study, Robitaille et al (Food Res Int (1993) 26, 365-369) showed that removal of sialic acid residues from κ-casein has no significant effect on the coagulation time but that the curd firmness decreased. WO02074097 describes the partial enzymatic deglycosylation of kappa-casein in the presence of sialidase (neuraminidase) to promote clotting of milk for cheese manufacture. However, none of these publications describes or suggests the use of sialidase for the production of sour dairy preparations.

Patent application WO2008/101893 describes the production of a novel sialidase from the filamentous fungus *Penicillium chrysogenum* and use thereof in food applications. The specific use of this sialidase for the production of sour dairy preparations has, however, not been described.

The current invention relates to a method of manufacturing yoghurt or drinking yoghurt with the use of a sialidase enzyme. The method allows the modification of the texture more advantageously and easily than known methods. In addition, the method allows a (significant) reduction in the added protein content or allows a reduction in the amount of added stabilizers or texturizers without any damage to or adverse effect to the texture of the yoghurt or drinking yoghurt.

This leads for the manufacture to lower raw material cost level than in the manufacture at a conventional protein level.

Without being bound by theory it is believed that in a casein micelle the sialic acid resides on the outside of the micelle, contributing to the overall charge of the casein micelle. When the sialidase removes the sialic acid from the casein micelle, it also removes negative charge from surface of the micelle, changing the colloidal nature of the casein micelle. With a different charge, pH-change-induced aggregation processes also change, and as a consequence a different type of aggregate can be formed on acidification. This turns out to be a stronger protein network after sialidase treatment - or an equally strong network with less protein. And this effect is independent of the way of acidification, either fermentative or chemical.

In a first embodiment, the invention provides a method for preparing a yoghurt or drinking yoghurt comprising adding a sialidase and an acidifying agent to a sialic acid comprising protein, allowing acidification to take place and optionally recovering the produced yoghurt or drinking yoghurt The term 'acidifying agent' refers to a microbiological starter or culture, a chemical acidifying agent or mixtures thereof. Acidifying may be performed by fermenting with at least one product specific culture and/or by using chemical acidifying agents, such as organic or inorganic acids. The microbial starter or culture may contain one or more different microbial species. More preferably the microbial starter or culture consists of one or more bacterial species, more preferably one or more lactic acid bacterial species.

Sour dairy preparations may be prepared with the use of bacteria, for example with lactic acid bacteria from the species *Lactococcus lactis ssp. cremoris* and *spp. lactis, Lactococcus lactis ssp. lactis biovar. diacetylactis, Leuconostoc sp., Lactobacillus* sp. such as *Lactobacillus helveticus, Lactobacillus casei, Lactobacillus rhamnosus, Lactobacillus acidophilus, Lactobacillus delbrueckii* and/or *Lactobacillus plantarum, Acetobacter orientalis, Streptococcus thermophilus, Bifidobacterium sp.* and/or *Pediococcus* species or combinations of a number of species. As an example yoghurt is typically prepared by inoculation of milk with *Lactobacillus delbrueckii subsp. bulgaricus* and *Streptococcus thermophilus* as starter cultures. As another example twarog is typically prepared by using *Lactococcus lactis ssp. cremoris, Lactococcus lactis ssp. lactis, Lactococcus lactis ssp. lactis biovar. diacetylactis, Leuconostoc sp.,* or *Streptococcus thermophilus.* The skilled person is, based on the knowledge of a particular product, capable of selecting the right type(s) of culture(s). The species in a starter culture may not be fully defined and may also contain fungi, like yeasts or filamentous fungi.

The skilled person is capable of selecting the most suitable microbiological starter or culture.

The chemical acidifying agent may be an acid, more preferably an organic acid, like citric, acetic, formic, propionic or lactic acid or another organic acid or combinations thereof. Alternatively, acidification may be performed using glucono-delta-lactone. With the exception of the addition of sialidase enzyme and an optional adjustment of the protein content of the protein source of the starting material (i.e. the material comprising sialic acid comprising protein, such as, but not limited to milk or reconstituted milk; the starting material is the material which is used to prepare the yoghurt or drinking yoghurt from), acidifying may take place in the same way as for corresponding yoghurt or drinking yoghurt products manufactured conventionally by the use of an acidifying agent suitable for each particular case and/or each particular product, and suitable reaction conditions. The treatment of the starting material (for example milk or reconstituted milk) with sialidase may occur before an optional heat treatment. The sialidase-treated milk can be heated before acidification. Alternatively, sialidase may be added after an optional heat treatment. Sialidase may then be used during acidification using an acidifying agent of choice. In a preferred embodiment, the sialidase treatment is performed before acidification, i.e. in a preferred embodiment the invention provides a method for preparing yoghurt or drinking yoghurt comprising adding a sialidase and an acidifying agent to a sialic acid comprising protein, allowing acidification to take place and optionally recovering the produced yoghurt or drinking yoghurt, wherein said sialidase is added before said acidifying agent is added and wherein said sialidase and said sialic acid comprising protein are incubated for a time sufficient to obtain free sialic acid and after obtaining sufficient free sialic acid said acidifying agent is added.

The protein source used in the manufacturing of a yoghurt or drinking yoghurt may be a sialic acid containing protein. A 'sialic acid comprising protein' is a protein, usually of animal origin, that contains one or more sialic acid groups attached to a peptide chain. Other sources of a sialidase comprising protein are plant, fungi, yeast and bacteria. Attachment of sialic acid to the peptide chain can be direct or via glycosyl groups. In general 'sialic acid comprising proteins' are glycosylated and contain α2-3 linked or α2-6 linked terminal sialic acids. The 'sialic acid comprising protein' may be a small peptide such as glycomacropeptide (GMP) or proteose peptone, or a larger protein such as kappa-casein. The sialic acid comprising protein is typically part of a starting material, i.e. the material which is used as a basis to prepare a yoghurt or drinking yoghurt from, such as, but limited to, milk or reconstituted milk.

Typically, the exact reaction conditions are selected based on the specific type of yoghurt or drinking yoghurt that is being produced. These conditions are chosen to allow acidification to proceed. At the same time, the conditions are preferably such that the applied sialidase is active for at least a certain amount of time to allow it to perform its reaction on the sialic acid comprising protein. The acidification of a typical sour dairy preparation like yoghurt, starts by the addition of the starter culture to pasteurized milk at 42 degrees Celsius, and incubation for 4-5 h. During this period the pH of the preparation will decrease from 6.5-6.7 to 4.0-4.5. Sialidase may be added at the beginning or during the acidification process.

When there is not sufficient time during the acidification process, e.g. when the acidification is performed by the addition of acid, the sialidase treatment is preferably performed prior to the acidification. Typically, sialidase treatment will take minimally 1 hour at elevated temperature like 37 degrees Celsius, or a longer period at a reduced temperature like 8 degrees Celsius. Obviously, the incubation period may be adjusted by increasing or decreasing the sialidase dosage. This also applies to processes where the sialidase needs to be inactivated by heat treatment. The heat treatment usually performed prior to milk fermentation for yoghurt (such as 5 minutes 90-95°C) is sufficient to inactivate the enzyme. In yet another preferred embodiment, the invention therefore provides a method for preparing a yoghurt or drinking yoghurt comprising adding a sialidase and an acidifying agent to a sialic acid comprising protein, allowing acidification to take place and optionally recovering the produced yoghurt or drinking yoghurt, comprising the steps of:
- incubating a suitable starting material (for example milk or reconstituted milk) with sialidase under conditions such as to obtain free sialic acid
- subjecting the sialidase-treated starting material to a heat treatment (for example 2-5 minutes at 90-95 degrees Celsius)
- adding an acidifying agent to the sialidase and heat treated starting material
- allowing acidification to take place and optionally recovering the produced yoghurt or drinking yoghurt.
Depending on the particular preparation which is prepared a recovering step might need to be included. As an example the production of yoghurt is described. Three basic types of yoghurt exist, according to its physical state in the retail container: set yoghurt, stirred yoghurt and drinking yoghurt. For set yoghurt the inoculated milk is fermented after being packed in a retail container. Stirred yoghurt is almost fully fermented in a fermentation tank before it is packed, the yoghurt gel being broken up during the stirring and pumping. If stirred yoghurt is produced, the produced yoghurt needs to be transferred to its final packaging (i.e. needs to be recovered). If set yoghurt is produced such a recovering will not be necessary. In other words whether or not a recovery step is necessary depends on the specific preparation produced. The skilled person is very well capable of determining whether or not a recovery step needs to be included.

For drinking yoghurt the process follows roughly along the same lines as the stirred yoghurt process. However after fermentation the yoghurt gel is broken and subjected to a substantial shear treatment that breaks down the structure completely. In order to obtain a stable product, a stabiliser such as pectin can be added, dissolved in another aqueous phase. The relative volume of this separate aqueous phase can be substantial, much more than 50 volume percent. This phase may consist of (acidified) water, or could contain other constituents such as flavours, fruit juice, sweeteners, fibres, further hydrocolloids and (modified) starches and so on. Drinking yoghurts can also be prepared from milk that is diluted with an aqueous phase (water, optionally with sweetener and other optional ingredients) before it is fermented. In a similar manner acidified milk drinks are prepared, where instead of a fermentation with lactic acid bacteria, acidification is done by adding a chemical acid, such as lactic acid, citric acid or any other type of food grade acid.

In one aspect of the invention, a fraction of the starting material may be treated separately using sialidase and added to the product before or after (preferably before) acidification. This fraction may first be processed further before addition to the final preparation. For example, sialidase may be used to treat skim milk before drying the sialidase-treated skim milk producing sialidase treated skim milk powder, which can be added to a preparation to produce a yoghurt or drinking yoghurt after acidification using an acidifying agent of choice. Or, a (pre-isolated) sialic acid comprising protein is treated with sialidase to produce a treated protein and use this protein in the preparation of a yoghurt or drinking yoghurt The invention therefore also provides a method for preparing a yoghurt or drinking yoghurt comprising adding a sialidase and an acidifying agent to a sialic acid comprising protein, allowing acidification to take place and optionally recovering the yoghurt or drinking yoghurt, wherein at least part of said sialic acid comprising protein is pre-incubated with said sialidase to obtain treated protein, optionally recovering the treated protein, further comprising adding the treated protein to a dairy starting material used to prepare said yoghurt or drinking yoghurt. Alternatively worded, the invention provides a method for preparing a yoghurt or drinking yoghurt from a sialic acid comprising protein comprising the steps of:
- incubating at least part of the sialic acid comprising protein with a sialidase to obtain treated protein
- optionally recovering the treated protein
- adding treated protein to a starting material suitable for preparing a yoghurt or drinking yoghurt from
- adding an acidifying agent
- allowing acidification to take place and optionally recovering the produced yoghurt or drinking yoghurt. Preferably, the said starting material comprises sialic acid comprising protein. Optionally, additional sialidase may be added before or during acidification.

The invention also provides a method for preparing a yoghurt or drinking yoghurt comprising adding a sialidase and an acidifying agent to a sialic acid comprising protein, allowing acidification to take place and optionally recovering the produced yoghurt or drinking yoghurt wherein said sialic acid comprising protein is present in a dairy starting material, optionally recovering the treated dairy starting material, drying the treated dairy starting material and adding said dried treated dairy starting material to a (non treated) diary starting material used to prepare said yoghurt or drinking yoghurt. Alternatively worded, the invention also provides a method for preparing a yoghurt or drinking yoghurt from a sialic acid comprising protein comprising the steps of:
- incubating a dairy starting material with sialidase to obtain a treated dairy starting material
- optionally recovering the treated dairy starting material
- optionally drying the treated dairy starting material
- adding said (optionally dried) treated dairy starting material to a (non treated) diary starting material
- adding an acidifying agent
- allowing acidification to take place and optionally recovering the produced yoghurt or drinking yoghurt. Preferably, the said starting material comprises sialic acid comprising protein. Optionally, additional sialidase may be added before or during acidification. Preferably the dairy starting material is milk or reconstituted milk.

In a method according to the invention, raw material milk as such or pre-processed and /or fractionated in the desired manner usually constitutes the protein source. In this context, a starting material in general and a raw material milk in specific refers, for example, to milk obtained from an animal, e.g. a cow, buffalo, sheep or a goat, as such or processed in various manners. Milk may be processed for instance by removing fat or lactose from it, resulting in fat-free, low-fat, lactose-free or low-lactose milk. In this context, raw material milk also refers to pre-processed or unprocessed milks used in the manufacture of yoghurt, viili and fermented milk, for example.

The milk used for the preparation of a yoghurt or drinking yoghurt may also be obtained from a pre-processed milk fraction. For example, the milk may be obtained by dissolving skim milk powder in a liquid (i.e. and obtain reconstituted milk) before treatment with sialidase and acidification with an acidifying agent of choice. In other words, the starting material preferably consists of milk, such as skim milk, whole milk, cream or any combination thereof. In further embodiments, the dairy starting material is prepared, totally or in part, from dried milk fractions, such as e.g. whole milk powder, skim milk powder, casein, caseinate, total milk protein or buttermilk powder, or any combination thereof. In other words, the starting material can be selected from skim milk, whole milk, cream or any combination thereof or is prepared totally or in part, from dried milk fractions, such as e.g. whole milk powder, skim milk powder, casein, caseinate, total milk protein or buttermilk powder, or any combination thereof.

The invention therefore provides a method for preparing a yoghurt or drinking yoghurt comprising adding a sialidase and an acidifying agent to a sialic acid comprising protein, allowing acidification to take place and optionally recovering the produced yoghurt or drinking yoghurt wherein said sialic acid comprising protein is present in a dairy starting material, such as milk or reconstituted milk. Preferably, said dairy starting material is low-fat, fat-free, low-protein or protein-free milk.

The milk may be fractionated for instance by means of chromatographic separation and/or micro, nano or ultra-filtering into fractions containing different amounts of different components, and the fractions may, in turn, be used for adjusting the protein content of the milk as such or as different mixtures thereof.

As described above, it has now surprisingly been found that the addition of sialidase enzyme in the process of making a yoghurt or drinking yoghurt allows a reduction in the protein content of yoghurt or drinking yoghurt or allows a reduction in the amount of stabilizers and/or texturizers, without any damage to or adverse effect on the texture of the preparation. Therefore, the use of sialidase can reduce the addition of extra protein to a sour dairy preparation without affecting the final texture of the yoghurt or drinking yoghurt. This is for example very useful in the preparation of low-fat or fat-free yoghurt, i.e. a yoghurt which is produced from skimmed or defatted milk. Since the fat content has a large influence on the texture of the final product, low-fat or fat-free yoghurts produced without any additions are thin. Since market preference is on highly structured sour dairy preparations, it is advantageous to fortify the milk before acidification. Such fortification is typically obtained by the addition of protein or texturizers like hydrocolloids. Due to the addition of sialidase the amount of added protein or texturizer (for example the amount of pectin, starch or gelatin) can be reduced without negatively affecting the characteristics of the final product.

Preferably, said reduction is at least 10%, 20% or 30%. More preferred is a reduction in added/amount of protein or texturizer or stabilizer of at least 40, 50 or 60%.

In respect of stabilizers and/or texturizers the following example is provided. Acidified milk products and drink yoghurts are typically prepared by using pectin to stabilise the protein flocs and prevent syneresis. An acidified milk beverage is for example prepared by adding a pectin+sugar solution to reconstituted milk or raw (optionally water diluted) milk. The resultant is mixed. The pH is brought to 3.8-4.2 by adding acids and/or fruit juice. The obtained mix is homogenized, heated to 90 degrees Celsius for 5 to 15 seconds, cooled to ambient temperature and filled aseptically. Adding a sialidase to the process (for example to the reconstituted milk or raw (optionally water diluted) milk) allows for a reduction in the amount of added pectin.

The invention therefore provides a method for preparing a yoghurt or drinking yoghurt comprising adding a sialidase and an acidifying agent to a sialic acid comprising protein, allowing acidification to take place and optionally recovering the produced yoghurt or drinking yoghurt wherein said sialic acid comprising protein is present in a dairy starting material, such as milk or reconstituted milk, wherein said dairy starting material has a reduced protein and/or fat content. Alternatively worded, the invention provides a method for preparing a yoghurt or drinking yoghurt comprising adding a sialidase and an acidifying agent to a sialic acid comprising protein, allowing acidification to take place and optionally recovering the produced yoghurt or drinking yoghurt wherein said sialic acid comprising protein is present in a dairy starting material having a reduced protein an/or fat content, such as reduced protein milk, low fat milk, fat free milk, reduced protein reconstituted milk, low fat reconstituted milk or fat free reconstituted milk.

A yoghurt or drinking yoghurt with 'reduced protein' may also encompass sour dairy preparations where less additional protein is added in the manufacturing process, compared to conventional yoghurt or drinking yoghurt. In this embodiment the treatment of sialic acid comprising protein with sialidase will improve the structure of the yoghurt or drinking yoghurt with reduced protein in such a way that no or reduced additional protein is required to reach an acceptable structure of the final yoghurt or drinking yoghurt.

The protein content for a yoghurt type product may be as low as 0.5% by weight. More preferably, the protein content is within the range of 2 to 3% weight. There is no actual upper limit for the protein content but in practice a 10% protein content will rarely be exceeded. In a preferred embodiment, the protein content will be between 0.5 - 10% weight.

In another embodiment of the invention, the method further comprises a step of decreasing the protein content of the protein source by the addition of liquid suitable for that purpose. In this case, the method comprises the following steps: addition of a liquid suitable for decreasing the protein content, such as milk permeate, whey, lactose fraction, a concentrate thereof or a mixture constituted by milk permeate, whey, lactose fraction and/or concentrates thereof, to a protein source, addition of sialidase enzyme and an acidifying agent to the protein source, acidifying and recovering the product obtained. An advantage of a method according to the invention is that it enables the utilization of milk having lower (added) protein content than in the use of the above-described, known methods.

In yet another embodiment, the invention provides a method for preparing a yoghurt or drinking yoghurt comprising adding a sialidase and an acidifying agent to a sialic acid comprising protein, allowing acidification to take place and optionally recovering the produced yoghurt or drinking yoghurt wherein said yoghurt or drinking yoghurt is a yoghurt or drinking yoghurt having at least one improved characteristic. Preferably, said at least one improved characteristic is selected from an improved structure, and improved texture, an increased viscosity, an improved mouth feel and a decreased syneresis.

The terms 'improved structure' and 'improved texture' refer to an increased quality of the appearance and feel of a product. With respect to the current invention 'improved structure' and 'improved texture' will generally be related to an increased viscosity of the final yoghurt or drinking yoghurt.

The term 'increased viscosity' refers to an increased resistance of a substance to flow. With respect to the current invention viscosity can be measured using methods described in the examples of this application, including the use of a Brookfield instrument or a rheometer, or any other conventional method to measure viscosity known in the art, such as a posthumus funnel or a Bostwick Consistometer.

The term 'mouthfeel' refers to the texture or structure of a substance as it is perceived in the mouth. Mouthfeel can be determined using a trained sensory panel or by a (larger) set of untrained people.

The term 'syneresis' refers to the process in which a gel contracts on standing and exudes liquid. Syneresis may be quantified by measuring the amount of exuded liquid compared to the total amount of the sour dairy preparation. Other methods to measure syneresis in sour dairy preparations have been described in the art (Amatayakul et al. (2006) Int. J. Dairy Technol. 59, 216-221).

Whether or not a certain characteristic is improved, is determined by comparing the particular characteristic of a yoghurt or drinking yoghurt obtained by a method of the invention with the same characteristic of a non-sialidase treated yoghurt or drinking yoghurt which is otherwise identically prepared.

The invention thus provides a method for preparing a yoghurt or drinking yoghurt comprising adding a sialidase and an acidifying agent to a sialic acid comprising protein, allowing acidification to take place and optionally recovering the produced yoghurt or drinking yoghurt wherein said yoghurt or drinking yoghurt is a yoghurt or drinking yoghurt having at least one improved characteristic compared to a non-sialidase but otherwise comparable prepared yoghurt or drinking yoghurt.

As described, the use of sialidase in the preparation of yoghurt or drinking yoghurt will lead to the reduction of syneresis. In an alternative embodiment, the invention therefore provides a method for reducing syneresis in a yoghurt or drinking yoghurt comprising adding a sialidase or a sialidase treated protein to a dairy product and otherwise producing said yoghurt or drinking yoghurt according to any known method. In case of yoghurt such a method would comprise acidification and optionally recovering of the sour dairy product.

With respect to the order in which a sialidase and an acidifying agent are added to a dairy starting material it is noted that they may be added at the same time (i.e. simultaneously) via the same or via two different entries. Moreover, it is possible to first add the sialidase and allow it to perform its action for a certain amount of time after which the acidifying agent is added, optionally after a heat step that denatures the whey protein, pasteurises the milk and inactivates the sialidase. It is furthermore possible to add the sialidase during acidification.

The invention thus provides a method for preparing a yoghurt or drinking yoghurt comprising adding a sialidase and an acidifying agent to a sialic acid comprising protein, allowing acidification to take place and optionally recovering the produced yoghurt or drinking yoghurt wherein said sialidase and said acidifying agent are added simultaneously or wherein said sialidase is added during acidification. Preferably, the sialidase may also be added before acidification.

In yet a further preferred embodiment, the sour dairy preparation produced according to any of the above described methods is yoghurt or drinking yoghurt.

Yoghurt can be either set or stirred yoghurt.

In an alternatively further preferred embodiment, the sour dairy preparation produced according to any of the above described methods is drinking yoghurt.

With respect to the sialidase the following information is provided.

Sialidases (neuraminidases, EC 3.2.1.18) hydrolyze the terminal, non-reducing, sialic acid linkage in glycoproteins, glycolipids, gangliosides, polysaccharides and synthetic molecules. Sialidases are common in animals of the deuterostomate lineage (Echinodermata through Mammalia) and also in diverse microorganisms that mostly exist as animal commensals or pathogens. Sialidases, and their sialyl substrates, appear to be absent from plants and most other metazoans. Even among bacteria, sialidase is found irregularly so that related species or even strains of one species differ in this property. Sialidases have also been found in viruses and protozoa.

Sialidase activity was detected in various human cells, tissues and body fluids. In general, mammalian sialidases are unstable and, in the case of membrane-bound enzymes, they become more labile when dissociated from the membrane. Sialidase activity is found in cytosol, lysosomes and plasma membranes of cells. Plasma membrane sialidases are usually referred as 'ganglioside sialidase' and their enzyme specificity is largely restricted to membrane gangliosides. Cytosolic and lysosomal sialidases hydrolyze glycoproteins and oligosaccharides. Human sialidases occur in a high molecular-mass complex with several other proteins, including cathepsin A and β-galactosidase. Multi-protein complexation is important for the *in vitro* integrity and catalytic activity of the sialidase

Sialidase is also found in a variety of bacterial strains, including *Clostridium, Vibrio, Corynebacterium, Bacteroides, Streptococcus, Pasteurella, Salmonella, Arthrobacter, Actinomyces,* and *Streptomyces* species. Microbial sialidases may be excreted into the extracellular milieu, membrane bound, periplasmic or cytoplasmic depending on the species in which they are expressed and their diverse function.

Bacterial sialidases show diverse substrate specificity with a pH optimum between 5-7. Substrate linkage specificity also differs among bacterial sialidases. In general there is a preference cleavage of α2-3 linked terminal sialic acids above the α2-6 isomer, apart from some exceptions.

Bacterial sialidases can be classified in two families depending on the molecular size and Ca²⁺ requirement. Sialidases with a molecular weight of around 42 kDa belong to a "small" sialidase family. They include enzymes from *Clostridium perfringens, C. sordelli, Salmonella typhimurium* and *Micromonospera viridifaciens* and do not require the cation for activity. Significant sequence homology has been observed among these enzymes and they share a similar tertiary structure. Sialidases with a higher molecular weight belongs to a "large" sialidase family for instance *Vibrio cholerae* and require Ca²⁺ for full activity. Sequence analysis reveals little homology with viral and "small" bacterial sialidase.

Many sialidase-producing non-pathogenic strains have been identified in faecal and oral isolates. In the human large intestine these bacteria belong to the enteric bacteria and comprise anaerobes, e.g. Bacteroides sp., *E.coli, Enterococcus faecalis* and a group of mucin oligosaccharide-degrading bacteria including *Ruminococcus* and *Bifidobacterium* sp, which can completely degrade the glycoproteins present in the mucus at the surface of the normal mucosa. The production of sialidase within the mucin-degrading group is constitutive and acts on sialo-glycoproteins and gangliosides.

Micro-organisms containing sialidases often live in contact with higher animals as hosts, for example as parasites. Here they may have a nutritional function enabling the scavenge of host sialic acids to use as a carbon source. For some microbial pathogens, sialidases are believed to act as virulence factors. Yet, the role of sialidases as factors in pathogenesis is controversial. On the one hand they confirm the impact of pathogenic microbial species like *Clostridium perfringens.* On the other hand, these enzymes are factors common in the carbohydrate catabolism of many non-pathogenic species, including higher animals. They do not, however, exert a direct toxic effect (Traving et al Cell Mol Life Sci (1998) 54, 1330-1349). Instead, their detrimental effect depends on the massive amount of enzyme that is released into the host together with other toxic factors upon induction by host sialic acids under non-physiological conditions.

In contrast to mammalian, bacterial and viral sialidases, fungal sialidases have been much less studied. However, recently a secreted sialidase from the filamentous fungus *Penicilium chrysogenum* has been over-expressed in the food-grade fungus *Aspergillus niger* and is available for use in food applications (WO08101893).

Preferably, a method according to the invention uses a bacterial or fungal sialidase.

In a preferred embodiment, the invention provides a method for preparing a yoghurt or drinking yoghurt comprising adding a sialidase and an acidifying agent to a sialic acid comprising protein, allowing acidification to take place and optionally recovering the produced yoghurt or drinking yoghurt wherein said sialidase is a fungal sialidase, more preferably a *Penicilium* sialidase and even more preferred a *Penicilium chrysogenum* sialidase. The yoghurt or drinking yoghurt in the examples are prepared with a sialidase from *Penicilium chrysogenum* which sialidase has been over-expressed in the food-grade fungus *Aspergillus niger* (WO08101893). Said *Penicilium chrysogenum* sialidase can equally well be produced in another (micro)organism. In yet another preferred embodiment, the invention provides a method for preparing a yoghurt or drinking yoghurt comprising adding a sialidase and an acidifying agent to a sialic acid comprising protein, allowing acidification to take place and optionally recovering the produced yoghurt or drinking yoghurt wherein said sialidase, preferably a fungal sialidase, has a pH optimum between pH 5.5 and pH 7.5. An example of such a sialidase is the *Penicilium chrysogenum* sialidase as described in WO08101893 and which sialidase is used herein within the experimental part.

The sialidase used in the invention may preferably be a fermentation produced enzyme. The fermentation produced sialidase may be obtained from bacteria, yeast or fungi. Preferably the fermentation produced sialidase is obtained from a filamentous fungus. More preferably the fermentation produced sialidase is from the fungus *Penicillium chrysogenum,* produced in the filamentous fungus *Aspergillus niger.*

The amount of sialidase used for the production of a yoghurt or drinking yoghurt may be adjusted according to the application, substrate concentration, incubation period, incubation temperature, incubation pH and other parameters that can fluctuate in a production process. Typically, the amount of sialidase used for the production of a yoghurt or drinking yoghurt is between 1 mg/l and 1 g/l.

During the manufacturing of a yoghurt or drinking yoghurt according to any of the herein described methods an additional ingredient may be added. Such an additional ingredient include a fruit preparation, flavour, protein, fat, probiotic bacteria, prebiotic and sugars which may be added to the sour dairy preparation before or after treatment with sialidase and before or after acidification using an acidifying agent of choice.

The method of the invention is suitable for the manufacture of flavoured and non-flavoured fatty and fat-free, and homogenized and non-homogenized fresh products. It is further suitable for the manufacture of lactose-free and low-lactose products. In addition to these steps, the method of the invention preferably comprises any other necessary manufacturing steps for accomplishing the end product, such as the addition of jam and/or sugar and/or heat- treatment (post-pasteurization).

In a yet another preferred embodiment, the invention provides a method for preparing a yoghurt or drinking yoghurt comprising adding a sialidase and an acidifying agent to a sialic acid comprising protein, allowing acidification to take place and optionally recovering the produced yoghurt or drinking yoghurt further comprising adding another enzyme, such as lactase, carboxypeptidase, protease, aminopeptidase, beta-galactosidase, esterase, transglutaminase, lipase, phospholipase, isomerase, oxidase or peroxidase or comprising adding a prebiotic, such as galacto oligosaccharide or fructose oligosaccharide, or comprising adding a probiotic culture, such as lactic acid bacteria or bifidobacteria or further comprising adding a flavour, texturizer or stabiliser. Examples of flavours are fruit, vanilla or sugar. Examples of stabilizers are starch, carrageenan, gums, pectin, gelatine or hydrolcolloids. Stabilizers may also be used for their texturizing properties. Additionally, dairy derived texturizers like whey protein concentrate or casein may be used. Also microparticulate protein may be used for texturizing.

In one preferred aspect the invention provides a method for preparing a yoghurt or drinking yoghurt comprising adding a sialidase and an acidifying agent to a sialic acid comprising protein, allowing acidification to take place and optionally recovering the produced yoghurt or drinking yoghurt, further comprising adding lactase. A preferred lactase is *K. lactis* lactase (for example Maxilact® from DSM).

In a different embodiment, the invention provides a yoghurt or drinking yoghurt obtainable according to a method as described herein. Preferably, any of such products is low fat or fat free. Even more preferred, any of such products is also low-lactose or lactose-free. Preferred combinations of these characteristics are: low fat and low lactose or low fat and lactose free or fat free and low lactose or fat free and lactose free.

Yoghurt can be either set or stirred yoghurt.

In another most preferred embodiment, the sour dairy preparation is drinking yoghurt.

Preferably, such a yoghurt or drinking yoghurt can be characterised by an improved structure, and improved texture, an increased viscosity, an improved mouth feel and a decreased syneresis preferably in the presence of a relative low protein level. A yoghurt or drinking yoghurt according to the invention can also be identified by detection of a sialidase or parts of a sialidase. Another possibility to distinguish a sialidase treated sour dairy preparation from a conventional sour dairy preparation is to measure the amount of free sialic acid in the final product. A sialidase treated sour dairy preparation will have a higher amount of free sialic acid, not bound to oligosaccharide or protein, in the final product. Content of free sialidase in sour dairy preparations can hereby increase more than 2-fold, preferably more than 5-fold, more preferably 10-fold or higher. Concentration of free sialic acid in the final preparation will thereby be higher than 0.1 mg/kg, preferably higher than 0.5 mg/kg, more preferably higher than 1 mg/kg, higher than 2 mg/kg, higher than 5 mg/kg, most preferably higher than 10 mg/kg.

In another aspect the invention provides the use of a kit of parts comprising a sialidase and an acidifying agent. Preferably such a composition or kit further comprises another enzyme or a prebiotic or a probiotic or a flavour or a texturizer or a stabiliser.

Examples of suitable enzymes are lactase, carboxypeptidase, protease, aminopeptidase, beta-galactosidase, esterase, transglutaminase, lipase, phospholipase, isomerase, oxidase or peroxidase. A preferred lactase is *K. lactis* lactase (for example Maxilact from DSM).

Examples of suitable prebiotics are galacto oligosaccharide or fructose oligosaccharide.

Examples of suitable probiotics are lactic acid bacteria or bifidobacteria.

Examples of a suitable flavour are fruit, vanilla or sugar.

Examples of a suitable stabilizer are starch, modified starch, carrageenan, gums, pectin, gelatine or other hydrocolloids.

Examples of suitable texturizers are the stabilizers, whey powder, skim-milk powder, milk protein concentrate powder, whey protein concentrate, casein or microparticulated protein.

The above described composition or kit of parts is very useful for preparing a sour dairy preparation. Alternatively, such a composition or kit of parts is used to reduce syneresis in a yoghurt or drinking yoghurt. Preferably said yoghurt or drinking yoghurt has decreased amounts of added proteins and/or is low lactose or lactose free. In a preferred embodiment, said kit further comprises instructions for use wherein said sialidase is added to a starting material comprising sialic acid comprising protein and allowed to incubate to obtain sialidase-treated protein and subsequently subjecting said sialidase-treated starting material to an acidification step (i.e. acidification is only allowed after sialidase treatment). If use is made of a starter culture to obtain acidification of the starting material, then said sialidase treatment is performed before adding said starter culture or said sialidase is added together or just after the addition of a starter culture.

The following examples illustrate the present invention. They are in no way intended to restrict the claims.

### EXAMPLES

### Examples 1

### Preparation of small scale fermented skim-milk using sialidase ZJW

Cloning, expression and purification of a sialidase enzyme from *Penicillium chrysogenum* has been described previously in WO08101893. This sialidase was named ZJW, produced from the filamentous fungus *Aspergillus niger* and used to obtain examples of the effect of sialidases in the production of sour dairy preparations. 40 gram of skim milk powder (Nilac, NIZO, the Netherlands) was dissolved in 400 ml of tap-water and distributed over four 100 ml capped bottles. The 4 bottles were treated as described in Table 1. Sialidase ZJW was added to bottles 2 and 3 in 10 and 50 mg/l respectively. Bottles 2 and 3 were subsequently incubated for 4 hours at 40 degrees Celsius. After this, all bottles were stored refrigerated during the night. Subsequently, all bottles were warmed to 40 degrees Celsius for 30 minutes, before addition of 0.004 U/I starter culture Delvo®-YOG CY-121 (DSM Food-Specialties, the Netherlands; CY=classic yoghurt culture; defined culture). In bottle 4 also 10 mg/l sialidase ZJW was added together with the starter culture.

**Table 1: Conditions used to prepare a sour dairy preparation from skim milk.**

| | add ZJW | incubate | store | add CY121 | add ZJW | incubate |
|---|---|---|---|---|---|---|
| 1 | 0 | 0 | O/N 4°C | .004 U/l | 0 | 5h 40°C |
| 2 | 10 mg/l | 4h 40°C | O/N 4°C | .004 U/l | 0 | 5h 40°C |
| 3 | 50 mg/l | 4h 40°C | O/N 4°C | .004 U/l | 0 | 5h 40°C |
| 4 | 0 | 0 | O/N 4°C | .004 U/l | 10 mg/l | 5h 40°C |

**Table 2: Results of the experiment described in table 1. Viscosity is strongly increased and syneresis is reduced when a sour dairy preparation is made with sialidase.**

| | final pH | % syneresis | Storage modulus (G') (Pa) | Yield point (G'=G") (% strain) |
|---|---|---|---|---|
| 1 | 4.17 | 18 | 16 | 70 |
| 2 | 4.21 | 11 | 51 | 100 |
| 3 | 4.18 | 1 | 127 | 130 |
| 4 | 4.2 | 10 | 28 | 80 |

After incubation for 5 hours at 40 degrees Celsius, a gel-like structure was formed in all bottles of acidified skim milk. The bottles were shaken to break the structure and pH was measured. No significant differences in pH were found between the different bottles (Table 2). All bottles were stored for three days at 4 degrees Celsius. After this time the viscosity was measured with a rheometer (Physica MCR 301 by Anton Paar, Graz, Austria). A strain sweep was performed, which is an oscillatory measurement at a fixed frequency (of 10 Hz) and increasing amplitude (from 0.01% to 1000%) at a fixed temperature of 5°C using a PP50 plate as spindle.

In table 2, storage modulus (G', expressed in Pascal, Pa) is a measure of the texture strength of the material; the higher, the stronger the material. It is obtained by putting an oscillatory movement on the sample and measuring the stress response. The yield point is the amplitude where the storage modulus becomes smaller than the loss modulus (G", the measure of the amount of stress that is dissipated by the material under the particular oscillatory movement). At that point onwards the material behaves more liquid like. This corresponds to the point where the microstructure of the material breaks up and can not cope with the stress put on it. The higher the yield point the higher the deformation that can be imposed on the material before it starts to flow.

A difference in texture was clearly visible by human perception and an increase in texture strength (G') was measured with the rheometer between the samples that were sialidase treated, and the control samples. Viscosity was significantly higher in bottles 2 and 4, and especially bottle 3. Also the yield point was higher when samples were treated with sialidase. Clearly, sialidase has a clear positive effect on texture strength.

After 2 weeks of storage at 4 degrees Celsius, syneresis was measured in all bottles, by dividing layer thickness (in cm) by total product height (in cm) and expressing it as percentage (Table 2). Results clearly show that treatment of sour dairy preparations with sialidase before or during acidification leads to an improved structure and reduced syneresis.

### Example 2

### Preparation of yoghurt using sialidase ZJW

1.5 liter homogenized semi-skimmed cow milk (1.5% fat, 3.5% protein) was heated for 30 min at 85°C in a water bath. The milk was rapidly cooled down to 42°C using iced water. The sialidase ZJW was added to the milk at a level of 10mg/L and incubated for 2 hours at 42°C. A control sample (without sialidase) was treated the same way. The milk then was inoculated with starter culture Delvo®-YOG CY-121 (DSM Food-Specialties, the Netherlands) at a level of 4U/1000L. For set yoghurt the inoculated milk was poured into its final package before fermentation at 42°C. For stirred and drinking the fermentation took place in 3 liter vessels. The process was stopped when the yoghurt reached pH4.5, which took about 4 hours. After fermentation the yoghurt was pumped through a homogenizer (APV Homogenizer Rannie) without pressure for stirred yoghurt production. For drinking yoghurt a homogenization at 100bar was performed. All yoghurts were stored in small containers at 5°C for a minimum of 4 days.

Relative viscosity was measured by means of a Brookfield DV-III Viscometer (Brookfield, USA) with a T-B spindle using the Helipath method (where a descending spindle penetrates clockwise through the yoghurt) at 30 rpm. By constantly measuring the resistance and calculating it back a value for viscosity is given in mPas (milli Pascal seconds).

Surprisingly, the viscosity was higher with the sialidase-treated samples than for the non-treated samples. The difference was best seen for the stirred yoghurt with a viscosity improvement of over 35% (Figure 1).

The samples were organoleptically assessed, and all assessors named the sialidase-treated stirred yoghurt sample as having more mouthfeel and clearly thicker.

### Example 3

### Measurement of sialic acid content

BioVision's Sialic Acid Assay Kit (CA, USA) provides a simple and convenient means of measuring free sialic acid in a variety of biological samples. The kit utilizes an enzyme coupled reaction in which free sialic acid is oxidized resulting in development of the Oxi-Red probe to give fluorescence (Ex/Em=535/587 nm) and absorbance (O.D.=570 nm). The kit measures sialic acid in the linear range of 0.1 to 10 nmol with a detection sensitivity ∼1 µM concentration.

Samples can be tested for free sialic acid or hydrolyzed to measure bound sialic acid as well using the kit of Biovision. For measurement of total sialic acid, first a hydrolysis at mild acid conditions should be performed. The sample from a sour dairy preparation is hydrolysed in 0.05 M H₂SO₄ at 80 degrees Celsius at a concentration of about 1 mg protein/ml. Samples for sialic acid determination are removed at appropriate times and stored at -20°C until analysis. After adjustment of the pH of the hydrolysate to approximately 5.5, and optional cleaning by deproteination or other methods that remove components that can disturb the measurement, sialic acid content is measured using the Biovision kit.

For measurement of free sialic acid, samples are optionally cleaned by deproteination or other methods that remove components that can disturb the measurement, and are directly measured using the Biovision kit.

Alternatively, sialic acid levels can be determined by using an HPLC method. An HPLC method to determine sialic acid levels is described by the Dionex Company (Dionex technical note 41; http://www.dionex.com/en-us/webdocs/5053-tn41.pdf). In short the following procedure is applied. Equipment: Dionex BioLC, consisting of a GS50 Quaternary HPLC pump, an AS50 Injector with Thermal Compartment, an ED50 electrochemical detector and a Chromeleon Chromatography data system, all products from Dionex (Amsterdam, the Netherlands); measuring conditions: CarboPac PA20 HPLC column, with Amino Trap guard column and Borate Trap pre-column, run at a flow rate of 0,5 ml/min, using an injection volume of 10 µL and a tray temperature 20°C, a column temperature of 30°C and a gradient consisting of 3 phases: Distilled water, 500 mM NaOH and 1M NaOAc in 0.1M NaOH.

A calibration curve was made with a sialic acid solution: 25 mg sialic acid (Fluka 01398) was accurately weighed in duplicate into a 25 ml volumetric flask and dissolved in distilled water. These solutions were diluted with water to a concentration of 1 to 7.5 mg/l and run in the HPLC as described above.

Milk or yoghurt samples were diluted 25 times with water and centrifuged for 5 min at 13000 rpm. From the clear supernatant about 0.5 ml was transferred into a Nanosep ultra filtration cartridge (10 KD) and centrifuged for at least 30 min at 14000 g, 250 µL of the clear filtrate was transferred into a 300 µL injection vial. Sialic acid levels were then determined as described above.

### Example 4

### Sialic acid release before or during yoghurt fermentation and impact on acidification rate

Yoghurt was made from full fat milk (1469 g, Albert Heijn) plus skim milk powder (Promex - Friesland-Campina, 31 g) to reach 4.35% protein, incubation was done at two levels (0.143 and 0.286 mg enzyme / g milk protein, corresponding to 10 or 20 mU/L) and before pasteurisation (4 hr 40°C) or during fermentation. Enzyme used was ZJB batch 0009.

Yoghurt was prepared as described in example 2, using Delvo-Yog CY-121 (160µL/L) as starter culture. Sialic acid levels in the yoghurts were measured by HPLC (see example 3) and are given in the table below. The pH value during acidification was measured by a pH meter; values are also given in the table below

**Table 3**

| Sample | Sialic acid (mg/kg) | pH during fermentation | | | | |
|---|---|---|---|---|---|---|
| | | 0 hrs | 2 hrs | 3 hrs | 4 hrs | 4.45 hrs |
| Control | 26,9 | 6.55 | 5.97 | 5.25 | 4.77 | 4.57 |
| Low sialidase before pasteurisation | 40,2 | 6.57 | 5.94 | 5.21 | 4.77 | 4.55 |
| High sialidase before pasteurisation | 94,1 | 6.58 | 5.93 | 5.26 | 4.75 | 4.58 |
| Low sialidase during fermentation | 70,0 | 6.57 | 5.94 | 5.25 | 4.73 | 4.57 |
| High sialidase during fermentation | 121 | 6.57 | 5.94 | 5.21 | 4.74 | 4.57 |

This proves that sialic acid can be hydrolysed in milk at neutral pH as well during yoghurt fermentation when the pH gradually drops. It also proves that sialidase treatment does not influence the rate of acidification.

### Example 5

### Milk incubation trials

Several milk sources were incubated with sialidase to determine the rate of sialic acid release. The milk sources were skim milk (Albert Heijn, the Netherlands, 4% protein); Skim Milk Powder (SMP; Promex - Friesland-Campina, standardised on 4% milk protein in water) and Milk Protein Concentrate (MPC; TMP Milei, Leutkirch, Germany), standardised on 4% milk protein in water). All milk sources were incubated at 30°C and milk also at 4°C. Incubation levels were 80 mU sialidase / liter milk except for one incubation of normal skim milk which was done at 20 mU/L. In the table the released sialic acid levels are given as determined by HPLC (see example 3), expressed in mg/L.

**Table 4**

| Time | Milk 30°C | Milk 30°C | SMP 30°C | MPC 30°C | Milk 4°C |
|---|---|---|---|---|---|
| (hours) | 20mU/L | 80 mU/L | 80mU/L | 80mU/L | 80 mU/L |
| 0 | 20.4 | 22.6 | 21,4 | 1,5 | 22.6 |
| 0.5 | 64.7 | | | | |
| 1 | 77.3 | 126 | 122 | 84 | |
| 2 | 85,0 | 138 | 147 | 101 | 111 |
| 4 | 101 | 151 | 157 | 114 | 121 |
| 6 | 116* | 162 | 171 | 126 | 128 |
| 24 | 153 | 186 | 198 | 166 | 143 |
| 48 | | | | | 156 |

| | | | | | |
|---|---|---|---|---|---|
| * at 8 hrs | | | | | |

This proves that in all milk sources sialic acid can be released in substantial amounts in a reasonable time frame.

### Example 6

### Yoghurt test

Four variants of yoghurt were made from fresh skim milk (NIZO, Ede the Netherlands, <0.1% fat) + low heat SMP (skim milk powder; batch BEO1007773/s 1-02-055, Ingredia France) in 2 or 5 kg batches.
1. Ref 4.35% protein (A as such and B held 5 hr at 30°C)
2. 4.35% protein with sialidase (23mU/L)
3. 3.9% protein with sialidase (21 mU/L)
4. 3.6% protein with sialidase (19mU/L)

The milk was incubated for 6hr at 30°C with sialidase (batch NID/ ZJW0007, 6.5mU/g milk protein), pasteurised at 92°C for 10 minutes cooled to 37°C and inoculated by using 0.004% Delvo®-YOG CY-121 and fermented approximately 7 hrs at 37°C, until a pH of 4.6 was reached. Then the products were manually stirred shortly and cooled to 25°C and subsequently broken by using a Power Gen shearing device (Fisher Scientific) on speed 1, head on 2 mm slit for 12 seconds, shearing the vat from bottom to top of the tank. The products were filled in polypropylene cups.

Directly after breaking the viscosity of the product was assessed using a Haake RV20 Rotavisco analyser with MV2p geometry at 64 s⁻¹ for 1 minute. The viscosity value as reported by the analyser after 10 seconds of shearing are given in the table below. Sialic acid levels were determined by HPLC (see example 3) on the milk products before fermentation.

**Table 5**

| Sample | Sialic acid content in final product (mg/L) | Viscosity (Haake) 25°C at 64s⁻¹, after 10", in mPa.s |
|---|---|---|
| 1A - 4.35% protein no enzyme no holding | 24 | 206 |
| 1B - 4.35% protein no enzyme | 25 | 238 |
| 2 - 4.35% protein with enzyme | 124 | 428 |
| 3 - 3.9 % protein with enzyme | 113 | 219 |
| 4 - 3.6 % protein with enzyme | 104 | 189 |

This table shows that directly after breaking under constant conditions the yoghurt made from sialidase treated milk has a higher viscosity than the reference (compare samples 1 and 2), and that yoghurt products made with sialidase treated milk but a lower milk protein level (samples 3 and 4) have a comparable viscosity as a reference with a higher protein level.

### Example 7

### Yoghurt test

In this test, yoghurt made from full fat milk treated with sialidase at a protein level of 3.9% was compared to yoghurt made from normal full fat milk at a protein level of 4.35%.

Skim milk powder (Promex - Friesland-Campina) was dissolved in commercial pasteurised full fat milk (3.5% fat, Albert Heijn, the Netherlands). This mixture was incubated for 18 hrs at 4°C followed by 5 hrs at 30°C in variations as given in the table below. After that the milk was pasteurised for 30 minutes at 85°C. Then the milk was brought to 42°C and inoculated with Delvo®-YOG CY221 (mildly EPS forming) until a pH of 4.8 or 4.6 (see table). The product was broken directly, without further cooling, by passing once through a high pressure homogeniser (Rannie, APV, Denmark) without pressure, filled in cups, and stored at 4°C.

The viscosity of the products was characterised by Brookfield viscositymeter after various times: 0 days (20°C), 3, 7, 21 days (all 4°C). Sialic acid levels of the milk before inoculation were determined by HPLC, see example 3. All details are given in the table below.

**Table 6**

| # | % protein | Enzym e [mU / L] | Break at pH | Sialic acid [mg / L] | pH at day 7 | Viscosity [Brookfield, mPa.s] at day | | | |
|---|---|---|---|---|---|---|---|---|---|
| | | | | | | 0 (20°C) | 3 | 7 | 21 |
| 1 | 4.35 | - | 4.6 | 26 | 4.29 | 3040 | 5113 | 7180 | 6755 |
| 2 | 3.9 | 80 | 4.8 | 188 | 4.43 | 3265 | 5230 | 6291 | - |
| 3 | 3.9 | 80 | 4.6 | 191 | 4.29 | 2276 | 4112 | 5080 | 5698 |
| 4 | 3.9 | 20 | 4.6 | 136 | 4.23 | 2668 | 5084 | 5182 | 6216 |

This table shows that after sialidase treatment the viscosity of the yoghurt with lower protein level matches the viscosity of the high-protein no-enzyme product. Also, it shows that the yoghurt can be broken at an earlier stage when acidification has not progressed so far, is possible. This would give an advantage in processing time as well, especially given that the last stage of acidification goes fairly slow: Where products 1, 3, and 4 took 5hr 30min to come to final pH, product 2 only took 4 hrs and 30 minutes.

### Example 8

### Drinking yoghurt test

Yoghurt was made from regular commercial skim milk (3.5% protein, Albert Heijn) in the same way as described in the previous example, except for the use of the culture Delvo®-YOG CY220 up to the point of breaking. The milk was incubated with enzyme 80mU sialidase per liter of milk using batch ZJW0006, first 24 hrs at 4°C then 5 hrs at 30°C, before heat treatment. The fermented product was first broken by manual stirring and after that pumped through a high pressure homogeniser (Rannie, APV, Denmark) at a pressure of 100 Bar, filled in bottles, and stored at 4°C. Sialic acid levels of the milk samples were determined by HPLC as described in example 3.

After 2 weeks the viscosity was determined by a Physica MCR301 rheometer (Anton Paar, Vienna, Austria), using a 17 ml cup and spindle setting. The sample was sheared at a shear rate of 20s⁻¹ for 1 minute, collecting 12 data points. The viscosity value given is an average triplicate measurement of these 12 points. Syneresis was determined by leaving the product to stand for 20 days in 50 ml closed tubes and recording the volume of the clear upper layer that occurs during this time, expressed as ml/50ml. All values are given in the table below.

**Table 7**

| | Sialic acid mg/L | Viscosity mPa.s | Syneresis in ml/50ml on day | | | |
|---|---|---|---|---|---|---|
| | | | 4 | 7 | 14 | 21 |
| Reference | 19 | 61 | 3 | 5 | 8 | 10 |
| With sialidase | 149 | 317 | 1 | 1 | 3 | 6 |

From this table it is clear that drinking yoghurt made from sialidase-treated milk is more viscous and syneresis is considerably less than the reference. A higher viscosity was clearly apparent sensorially as tested in a panel of 8 persons.

### Example 9

### Drinking yoghurt

A next set of drinking yoghurts was made as follows: Skim milk (Albert Heijn, 3.5% protein) was diluted to 3.2 or 2.6% protein by water, one batch of each was incubated by sialidase (0.572 mg enzyme / g milk protein, batch ZJW0006 that contained 8.5 mg enzyme / ml solution). The four batches of milk were converted to yoghurt by Delvo®-YOG CY220 at 42°C. The yoghurt was broken by manual stirring, pectin solution (Genu Pectin YM 115-H, CPKelco, Copenhagen, Denmark) was added to a final level of 0.3% (w/v) and homogenised at 100 Bar by a high pressure homogeniser (Rannie, APV, Denmark).

Sialic acid levels of the milk samples were determined by HPLC as described in example 3. Viscosity was determined as described in example 8.

**Table 8**

| | Protein level (w/v %) | Sialic acid mg/L | Viscosity Pa.s |
|---|---|---|---|
| Reference | 2.6 | 18 | 0.14 |
| With enzyme | 2.6 | 134 | 0.38 |
| Ref with pectin | 2.6 | 18 | 1.26 |
| With enzyme + pectin | 2.6 | 134 | 1.51 |
| Reference | 3.2 | 21 | 0.48 |
| With enzyme | 3.2 | 151 | 0.52 |
| Ref with pectin | 3.2 | 21 | 1.93 |
| With enzyme + pectin | 3.2 | 151 | 2.33 |

From this table it is clear that drinking yoghurt made from sialidase-treated milk is more viscous, an effect which is independent of pectin present. A small sensory test proved that products with sialidase and pectin were perceived having a fuller mouthfeel. Also less syneresis was apparent after sialidase treatment (not quantified).

### Example 10

### Set yoghurt

Set yoghurt was prepared from full fat milk (3.5% protein, Albert Heijn) according to the same process as described in example 7. For the sialidase treatment the milk was incubated with sialidase (0.572 mg enzyme / g milk protein, batch ZJW0006 that contained 8.5 mg enzyme / ml solution) for 24 hrs followed by 5 hrs at 30°C. This resulted in milk with 156 mg sialic acid / L, whereas the reference had 20 mg / L. Sialic acid levels of the milk samples were determined by HPLC as described in example 3. After inoculation the milk was filled in cups, closed and left in a stove at 42°C for 5 hrs. After that the product was stored at 4°C. On day 7 the firmness was assessed by texture analysis (SMS TA-X2 Stable MicroSystems Godalming, Surrey, UK) using a half inch probe and a speed of 1 mm/s, penetrating to a depth of 10 mm. The firmness in grams is recorded at that depth. The control had a firmness of 37g; with average of 4 samples of sialidase-treated milk 38g.

## Claims

1. A method for preparing yoghurt or drinking yoghurt comprising adding a sialidase and an acidifying agent to a sialic acid comprising protein, allowing acidification to take place and optionally recovering the produced yoghurt or drinking yoghurt.

2. A method according to claim 1, wherein at least part of said sialic acid comprising protein is pre-incubated with said sialidase to obtain treated protein, optionally recovering the treated protein, further comprising adding the treated protein to a dairy starting material used to prepare said yoghurt or drinking yoghurt.

3. A method according to claim 2 wherein said sialic acid comprising protein is present in a dairy product, optionally recovering the treated dairy product, drying the treated dairy product and adding said dried treated dairy product to a dairy starting material used to prepare said yoghurt or drinking yoghurt.

4. A method according to claim 1, wherein said sialic acid comprising protein is present in a dairy product, such as milk or reconstituted milk.

5. A method according to claim 4, wherein said dairy product has a reduced protein and/or fat content.

6. A method according to any one of claims 1 to 5, wherein said yoghurt or drinking yoghurt is a yoghurt or drinking yoghurt having at least one improved characteristic.

7. A method according to claim 6, wherein said at least one improved characteristic is selected from an improved structure, an improved texture, an increased viscosity, an improved mouth feel and a decreased syneresis.

8. A method according to any one of claims 1 or 4-7, wherein said sialidase and said acidifying agent are added simultaneously to said sialic acid comprising protein or wherein said sialidase is added during acidification.

9. A method according to any one of claims 1 or 4-7, wherein said sialidase is added to and incubated with a sialic acid comprising protein before adding an acidifying agent and before allowing acidification to take place.

10. A method according to any one of claims 1 to 9, wherein said yoghurt is set yoghurt or stirred yoghurt.

11. A method according to any one of claims 1 to 10, wherein said sialidase is a bacterial or fungal sialidase.

12. A method according to claim 11, wherein said sialidase is a fungal sialidase, preferable a *Penicillium* sialidase, more preferably a *Penicillium chrysogenum* sialidase.

13. A method according to any one of claims 1 to 12 further comprising adding another enzyme, such as lactase, carboxypeptidase, protease, aminopeptidase, beta-galactosidase, esterase, transglutaminase, lipase, phospholipase, isomerase, oxidase or peroxidase or comprising adding a prebiotic, such as galacto oligosaccharide or fructose oligosaccharide, or comprising adding a probiotic culture, such as lactic acid bacteria or bifidobacteria or further comprising adding a flavour, texturizer or stabiliser.

14. A yoghurt or drinking yoghurt obtainable according to a method as claimed in any one of claims 1 to 13.

15. Use of a kit of parts for preparing yoghurt or drinking yoghurt, wherein said kit of parts comprises a sialidase and an acidifying agent.

16. Use according to claim 15, wherein said kit of parts further comprises another enzyme or a prebiotic or a probiotic or a flvour or a texturizer or a stabiliser.

## Patentansprüche

1. Verfahren zum Herstellen von Joghurt oder Trinkjoghurt, umfassend Zugeben einer Sialidase und eines Säuerungsmittels zu einem Sialinsäure-umfassenden Protein, Ermöglichen, dass Säuerung abläuft, und gegebenenfalls Abtrennen des hergestellten Joghurts oder Trinkjoghurts.

2. Verfahren gemäß Anspruch 1, wobei wenigstens ein Teil des Sialinsäure-umfassenden Proteins mit der Sialidase vorinkubiert wird, um behandeltes Protein zu erhalten, gegebenenfalls Abtrennen des behandelten Proteins, ferner umfassend Zugeben des behandelten Proteins zu einem Milchprodukt-Ausgangsmaterial, das zum Herstellen des Joghurts oder Trinkjoghurts verwendet wird.

3. Verfahren gemäß Anspruch 2, wobei das Sialinsäureumfassende Protein in einem Milchprodukt enthalten ist, gegebenenfalls Abtrennen des behandelten Milchprodukts, Trocknen des behandelten Milchprodukts und Zugeben des getrockneten behandelten Milchprodukts zu einem Milchprodukt-Ausgangsmaterial, das zum Herstellen des Joghurts oder Trinkjoghurts verwendet wird.

4. Verfahren gemäß Anspruch 1, wobei das Sialinsäureumfassende Protein in einem Milchprodukt enthalten ist, wie z. B. in Milch oder rekonstituierten Milch.

5. Verfahren gemäß Anspruch 4, wobei das Milchprodukt einen verringerten Protein- und/oder Fettgehalt aufweist.

6. Verfahren gemäß einem der Ansprüche 1 bis 5, wobei das Joghurt oder Trinkjoghurt ein Joghurt oder Trinkjoghurt mit wenigstens einem verbesserten Merkmal ist.

7. Verfahren gemäß Anspruch 6, wobei das wenigstens eine verbesserte Merkmal ausgewählt ist aus einer verbesserten Struktur, einer verbesserten Textur, einer erhöhten Viskosität, einem verbesserten Mundgefühl und einer verringerten Synärese.

8. Verfahren gemäß einem der Ansprüche 1 oder 4-7, wobei die Sialidase und das Säuerungsmittel gleichzeitig zu dem Sialinsäure-umfassenden Protein zugegeben werden oder wobei die Sialidase während der Säuerung zugegeben wird.

9. Verfahren gemäß einem der Ansprüche 1 oder 4-7, wobei die Sialidase zu einem Sialinsäure-umfassenden Protein zugegeben und damit inkubiert wird, bevor ein Säuerungsmittel zugegeben wird und bevor das Ablaufen von Säuerung ermöglicht wird.

10. Verfahren gemäß einem der Ansprüche 1 bis 9, wobei das Joghurt ein festes Joghurt oder gerührtes Joghurt ist.

11. Verfahren gemäß einem der Ansprüche 1 bis 10, wobei die Sialidase eine bakterielle oder fungale Sialidase ist.

12. Verfahren gemäß Anspruch 11, wobei die Sialidase eine fungale Sialidase ist, vorzugsweise eine *Penicillium-Sialidase,* bevorzugter eine *Penicillium-chrysogenum*-Sialidase*.*

13. Verfahren gemäß einem der Ansprüche 1 bis 12, ferner umfassend Zugeben eines weiteren Enzyms, wie z. B. Lactase, Carboxypeptidase, Protease, Aminopeptidase, beta-Galactosidase, Esterase, Transglutaminase, Lipase, Phospholipase, Isomerase, Oxidase oder Peroxidase, oder umfassend Zugeben eines Präbiotikums, wie z. B. Galactooligosaccharid oder Fructoseoligosaccharid, oder umfassend Zugeben einer probiotischen Kultur, wie z. B. Milchsäurebakterien oder Bifidobakterien, oder ferner umfassend Zugeben eines Aromas, Texturierungsmittels oder Stabilisators.

14. Joghurt oder Trinkjoghurt, erhältlich gemäß einem Verfahren gemäß einem der Ansprüche 1 bis 13.

15. Verwendung eines Kits zum Herstellen von Joghurt oder Trinkjoghurt, wobei das Kit eine Sialidase und ein Säuerungsmittel umfasst.

16. Verwendung gemäß Anspruch 15, wobei das Kit ferner ein weiteres Enzym oder ein Präbiotikum oder ein Probiotikum oder ein Aroma oder ein Texturierungsmittel oder einen Stabilisator umfasst.

## Revendications

1. Méthode de préparation d'un yaourt ou d'un yaourt à boire, comprenant les étapes consistant à ajouter une sialidase et un agent acidifiant à une protéine comprenant de l'acide sialique, laisser l'acidification avoir lieu et éventuellement récupérer le yaourt ou le yaourt à boire produit.

2. Méthode selon la revendication 1, dans laquelle au moins une partie de ladite protéine comprenant de l'acide sialique est préincubée avec ladite sialidase afin d'obtenir une protéine traitée, éventuellement récupérer la protéine traitée, comprenant en outre l'addition de la protéine traitée à une matière première laitière utilisée pour préparer ledit yaourt ou yaourt à boire.

3. Méthode selon la revendication 2, dans laquelle ladite protéine comprenant de l'acide sialique est présente dans un produit laitier, éventuellement récupérer le produit laitier traité, sécher le produit laitier traité et ajouter ledit produit laitier traité et séché à une matière première laitière utilisée pour préparer ledit yaourt ou yaourt à boire.

4. Méthode selon la revendication 1, dans laquelle ladite protéine comprenant de l'acide sialique est présente dans un produit laitier, tel que du lait ou du lait reconstitué.

5. Méthode selon la revendication 4, dans laquelle ledit produit laitier possède une teneur réduite en protéines et/ou en matières grasses.

6. Méthode selon l'une quelconque des revendications 1 à 5, dans laquelle ledit yaourt ou yaourt à boire est un yaourt ou yaourt à boire présentant au moins une caractéristique améliorée.

7. Méthode selon la revendication 6, dans laquelle ladite au moins une caractéristique améliorée est choisie parmi une structure améliorée, une texture améliorée, une viscosité accrue, une sensation améliorée en bouche et une synérèse réduite.

8. Méthode selon l'une quelconque des revendications 1 ou 4-7, dans laquelle ladite sialidase et ledit agent acidifiant sont ajoutés simultanément à ladite protéine comprenant de l'acide sialique ou où ladite sialidase est ajoutée lors de l'acidification.

9. Méthode selon l'une quelconque des revendications 1 ou 4-7, dans laquelle ladite sialidase est ajoutée à ladite protéine comprenant de l'acide sialique et incubée avec celle-ci, avant l'addition d'un agent acidifiant et avant de laisser l'acidification avoir lieu.

10. Méthode selon l'une quelconque des revendications 1 à 9, dans laquelle ledit yaourt est un yaourt ferme ou un yaourt brassé.

11. Méthode selon l'une quelconque des revendications 1 à 10, dans laquelle ladite sialidase est une sialidase bactérienne ou fongique.

12. Méthode selon la revendication 11, dans laquelle ladite sialidase est une sialidase fongique, préférablement une sialidase de *Penicillium,* plus préférablement une sialidase de *Pénicillium chrysogenum.*

13. Méthode selon l'une quelconque des revendications 1 à 12, comprenant en outre l'addition d'une autre enzyme, telle qu'une lactase, une carboxypeptidase, une protéase, une aminopeptidase, une bêta-galactosidase, une estérase, une transglutaminase, une lipase, une phospholipase, une isomérase, une oxydase ou une peroxydase, ou comprenant l'addition d'un prébiotique, tel qu'un galacto-oligosaccharide ou un fructo-oligosaccharide, ou comprenant l'addition d'une culture probiotique, telle qu'une bactérie lactique ou un *Bifidobacterium,* ou comprenant en outre l'addition d'un arôme, d'un agent de texture ou d'un stabilisant.

14. Yaourt ou yaourt à boire pouvant être obtenu selon une méthode selon l'une quelconque des revendications 1 à 13.

15. Utilisation d'un kit de parties pour la préparation d'un yaourt ou d'un yaourt à boire, dans laquelle ledit kit de parties comprend une sialidase et un agent acidifiant.

16. Utilisation selon la revendication 15, dans laquelle ledit kit de parties comprend en outre une autre enzyme ou un prébiotique ou un probiotique ou un arôme ou un agent de texture ou un stabilisant.
